(19) Europäisches Patentamt
European Patent Office
Office européen des brevets

(11) Publication number: **0 039 060**
**B1**

(12) **EUROPEAN PATENT SPECIFICATION**

(45) Date of publication of patent specification: **06.08.86**

(51) Int. Cl.$^4$: **C 07 H 15/24**

(21) Application number: **81103104.6**

(22) Date of filing: **24.04.81**

(54) **A new method for preparation of anthracycline derivatives.**

(30) Priority: **26.04.80 JP 55974/80**

(43) Date of publication of application:
**04.11.81 Bulletin 81/44**

(45) Publication of the grant of the patent:
**06.08.86 Bulletin 86/32**

(84) Designated Contracting States:
**BE CH DE FR GB IT LI LU NL SE**

(56) References cited:
**EP-A-0 014 853**
**EP-A-0 022 515**
**GB-A-2 002 754**
**US-A-3 803 124**

**CHEMICAL ABSTRACTS, vol. 92, no. 17, April 28, 1980, page 629, abstract 147129g Columbus, Ohio, USA D. HORTON, "Adriamycin analogs hydroxylated at C-3':synthesis and antitumor activity"**

**The file contains technical information submitted after the application was filed and not included in this specification**

(73) Proprietor: **Zaidanhojin Biseibutsu Kagaku Kenkyukai**
**14-23 Kamiosaki 3-chome**
**Shinagawa-ku Tokyo (JP)**

(72) Inventor: **Hamao, Umezawa**
**23 Toyotamakita 4-chome**
**Nerima-ku Tokyo (JP)**
Inventor: **Tomio, Takeuchi**
**5-1-11 Higashigotanda**
**Shinagawa-ku Tokyo (JP)**
Inventor: **Hiroshi, Naganawa**
**3-17 Denenchofuhoncho**
**Ohta-ku Tokyo (JP)**
Inventor: **Kuniaki, Tatsuta**
**26-7 Matsunoki 2-chome**
**Suginami-ku Tokyo (JP)**

(74) Representative: **Deufel, Paul et al**
**Patentanwälte Müller-Boré, Deufel, Schön, Hertel Lewald, Otto Isartorplatz 6**
**Postfach 26 02 47**
**D-8000 München 26 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 039 060 B1

**Description**

This invention relates to novel anthracycline derivatives, their preparation and their conversion to certain anthracycline glycosides which have known antitumor activity.

It is known from European Patent Application 80100352.6 (EP—A—14853) that anthracycline glycosides of the formula (III);

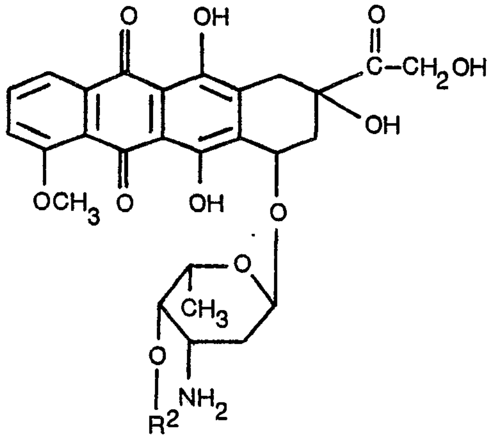

(III)

wherein $R^2$ is tetrahydropyran-2-yl, 6-acetoxymethyltetrahydropyran-2-yl, 6-methoxytetrahydropyran-2-yl, 6-carbomethoxytetrahydropyran-2-yl, tetrahydrofuran-2-yl, 1-methoxyethyl, 1-ethoxyethyl, 1-butyloxyethyl, 1-isobutyloxyethyl, 1-(6-methylheptyloxy)ethyl or cyclohexyloxyethyl group, have effective antitumor activity, and that these anthracycline glycosides can be synthesized using four steps from the starting material, daunomycin that is: (1) halogenation, especially bromination, of daunomycin with a halogen, especially bromine, to obtain the C—14 position halogenated, especially brominated, daunomycin derivatives of the formula (VII) (2) reacting said C—14 position brominated daunomycin with a compound of the formula $R^3$ COOK, for example, in acetone, wherein $R^3$ is a $C_1$—$C_6$ alkyl group or benzyl group, K is an alkali metal to obtain a compound of the formula (IV)

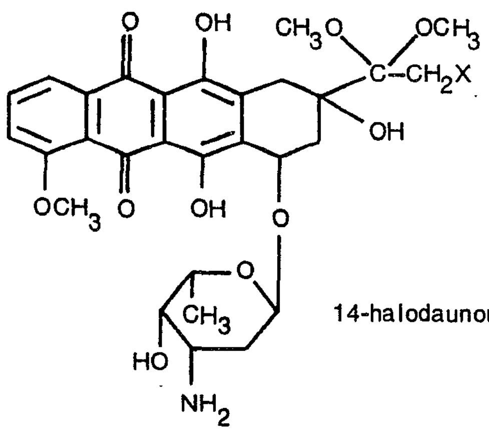

(VII)

14-halodaunomycinmethylketal

wherein X is a halogen atom, especially a bromine atom,

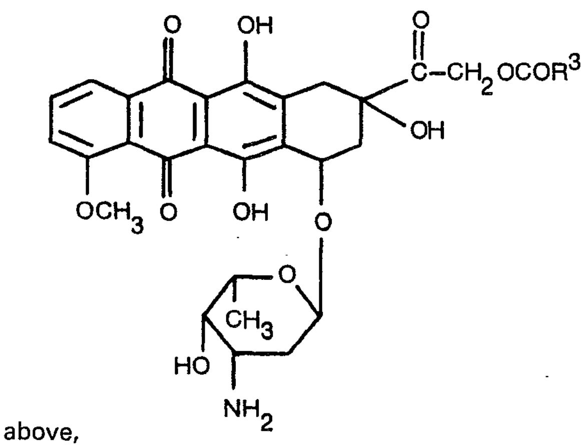

(IV)

wherein $R^3$ is as defined above,

(3) reacting the resulting compound (IV) with a dihydrofurane derivatives, dihydropyrane, a dihydropyrane derivatives or a vinylether derivatives, to obtain C—4′ position substituted derivatives of the formula (VI);

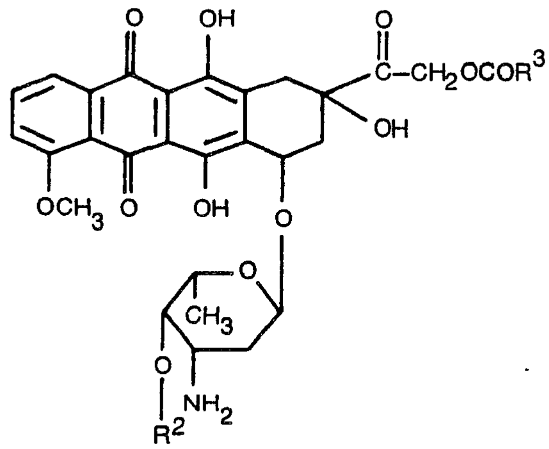

(VI)

wherein $R^2$, $R^3$ have the same meaning as defined above and (4) hydrolysing the resulting compound (VI) with an alkali in a lower alcohol or aqueous acetone to obtain C—14 position desacylated product of the formula (III).

In step 1 of this prior art process we have now found that there are simultaneously obtained both a compound of the formula (VII) mentioned above and a compound of the formula (I) as shown below.

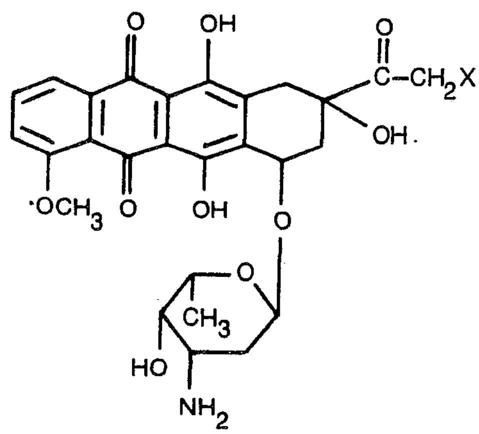

(I)

We considered this to result in a low yield of the final product (III), because it is synthesized from a mixture of compound (1) and (VII) with compound (I) being regarded as an impurity.

We have now found that C—14 position halogenation, especially bromination, of daunomycin (i.e. step 1 above) using a lower quantity of methanol than in said prior art process decreases the yield of (VII) and so that compound (I) is obtained as the main product.

We have also found that the compound (VII) is converted into the compounds (I) by acetone treatment, and that compounds (I) can be converted using a hydroxyl reagent into novel compounds of the formula (II);

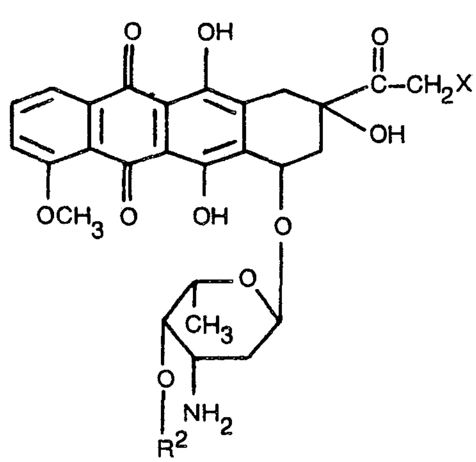

(II)

wherein X is a halogen atom, especially a bromine atom and $R^2$ is 6-substituted tetrahydropyran-2-yl such as 6-acetoxymethyltetrahydropyran-2-yl, 6-methoxytetrahydropyran-2-yl, 6-carbomethoxytetrahydropyran-2-yl; tetrahydrofuran-2-yl; alkyloxyethyl such as 1-methoxyethyl, 1-ethoxyethyl, 1-butyloxyethyl, 1-isobutyloxyethyl, 1-(6-methylheptyloxy)ethyl or cyclohexyloxyethyl group; the said hydroxyl reagents being dihydropyrane, substituted dihydropyranes, especially 2-acetoxymethyl-3,4-dihydro-2H-pyrane, 2-methoxy-3,4-dihydro-2H-pyrane and 2-carbomethoxy-3,4-dihydro-2H-pyrane, dihydrofurane, and vinyl ether derivatives, especially methylvinylether, ethylvinylether, butylvinylether, isobutylvinylether, 6-methylheptylvinylether and cyclohexylvinylether. These novel compounds are extremely useful as they are easily hydrolyzed by treatment with an alkali in a lower alcohol or aqueous acetone to obtain the antitumor compounds (III), which are the product of the abovementioned prior art process. This is surprising because ketal halo compounds obtained from compounds (VII) ar not easily hydrolyzed into hydroxyl compounds of the formula (III) by the same treatment.

US—A 3803124 is concerned with the preparation of adriamycin from daunomycin and relates only to hydroxylation at the C—14 position of daunomycin *inter alia* by bromination followed by hydrolysis. Substitution at the C—4′ position is not involved. GB—A 2002754 discloses the substitution of daunomycin or adriamycin with tetrahydropyranyl group(s) at the C—14 and/or C—4′ position and optional elimination of any unwanted tetrahydropyranyl group. Halogenation is not involved. Chemical Abstracts Vol 92, 1980, page 629, 92:147129g is concerned with the preparation of 3′-hydroxyadriamycin and involves only compounds having an hydroxy or acetate group at the C—3′ position.

Thus none of these disclosures contemplates the novel compounds of the present invention, their preparation or the preparation of the antitumor glycosides (III) therefrom.

In one aspect therefore this invention comprises the novel and useful compounds of formula (II) given above and its preparation from compounds (I), from compounds (VII) and ultimately from daunomycin.

In another aspect this invention comprises the production of the antitumor products (III) from novel compounds (II). The process aspects of this invention can also be regarded overall as a process for preparing antitumor compounds (III) from daunomycin by a three step process in improved (possibly by more than 16%) yield. This is in contrast to the four step prior art process. The process of the invention does not need the etherification of the C—4′ position of 14-halodaunomycin.

In each case reference to compounds (I), (VII), (II) and (III) is to be taken as a reference to their acid addition salts also.

In the practice of this invention, a daunomycin acid addition salt such as daunomycin hydrochloride in dry methanol can be brominated with bromine in methylene chloride in the presence of methyl orthoformate or dioxane and then the reaction mixture can be poured into dry ether. The treatment of the resulting precipitates with acetone gives the compounds represented by formula (I).

These compounds (I) can be converted to the 4′-substituted derivatives of formula (II) by treatment with the appropriate hydroxyl reagent to effect pyranylation, furanylation or etherification at C—4′ position. The resulting 4′-substituted derivatives of formula (II) can then be hydrolysed to the final product of formula (III).

In the halogenation (preferably bromination) at C—14 position of daunomycin of the formula (V) with halogen (preferably bromine) in inert organic solvent it is important to use a lesser quantity of methanol than in the prior art process so as to favour formation of compounds (I) over compounds (VII). The treatment of compounds (I) with dihydropyrane, dihydrofurane, their derivatives or alkylvinyl ether can be carried out in the presence of an acid catalyst such as sulfonates (for example, p-toluenesulfonate and camphorsulfonate) in inert organic solvent such as dimethylformamide to provide the 4′-substituted derivatives represented by formula (II). The hydrolysis (dehalogenation) of these compounds (II) at position 14 can be carried out in aqueous acetone or in lower alcohol.

The process steps of the present invention are industrially more advantageous than those of the prior art process, because the ketal-type (VII) of the halogenation product is least formed and the one step-less process results in improved yield and purity of the aimed products.

As a typical 4′-substitute tetrahydrofuran-2-yl is described in the Examples given below. However, as is detailed in European Patent Application 80100352.6, dihydropyrane compounds, dihydrofurane compounds and alkylvinyl ether compounds providing 6-substituted tetrahydropyran-2-yl such as 6-methoxytetrahydropyran-2-yl, 6-carbomethoxytetrahydropyran-2-yl and 6-acetoxytetrahydropyran-2-yl; tetrahydrofuran-2-yl and alkyloxyethyl such as 1-methoxyethyl, 1-ethoxyethyl, 1-butyloxyethyl, 1-isobutyloxyethyl, 1-(6-methylheptyloxy)ethyl and cyclohexyloxyethyl are also desirably employed for 4′ substitution.

For tetrahydropyranylation, tetrahydrofuranylation and etherification (these can collectively be called etherification in a broad sense), benzene, toluene, dimethylformamide(DMF), tetrahydrofurane(THF), dimethylsulfoxide(DMSO), dioxane and acetonitrile are conveniently used as anhydrous solvent solely or as mixtures. As the acid catalyst, sulfonates and particularly aromatic sulfonates such as p-toluenesulfonate, benzenesulfonate and DL-camphorsulfonate are preferable. However other common acid catalysts are also useful. Preferred combinations of the acid catalysts with the solvents are p-toluenesulfonate in anhydrous DMF, p-toluenesulfonate in a mixture of anhydrous DMSO and anhydrous THF or in a mixture of anhydrous DMSO and anhydrous dioxane, and DL-camphorsulfonate in anhydrous DMF. The etherification step can usually be completed in 20 minutes — 50 hours at room temperature.

The hydrolysis of 14-halodaunomycin derivatives in a water-miscible solvent such as lower alcohol (for example methanol and ethanol) and aqueous acetone can be carried out at room or slightly elevated temperature with alkali such as aqueous sodium hydroxide, while the extent of dehalogenation can be monitored by thin layer chromatography.

The products are obtained in the free base form or the acid addition salt form depending on the starting material employed. If desired, the free base can be converted to the acid addition salt by usual methods of salt formation or the acid addition salt can be recovered in the free base form.

The tetrahydropyranyl, tetrahydrofuranyl or alkoxyethyl derivatives obtained by the etherification in the present invention are a diastereomic mixture of isomers *a* and *b*. Thus they can be separated into individual isomers and purified at room temperature by conventional methods of purification for anthracycline glycosides.

For example, after solid matters are removed by filtration, the filtrate containing the desired products can be concentrated to dryness to give crude powder. The crude powder can then be subjected to purification for example by alumina or silica gel column chromatography or thin layer chromatography. The diastereomers *a* and *b* of the 4'-O-substituted derivatives seem to have absolute configurations R and S at the chiral center, because the two isomers differ in the chemical shift of the methine protons corresponding to the chiral center.

The final compounds of formula (III) according to this invention process can be obtained in the yield based on starting daunomycin as shown below in Table 1.

Some embodiments of the products and processes of the invention are described hereinafter in the Examples which are given for illustration only and are not to be construed as limiting the invention in any way.

### Example 1

4'-O-Tetrahydropyranyladriamycins *a* and *b*

14-Bromodaunomycin hydrochloride (6.0 g) is dissolved in 100 ml of anhydrous dimethylformamide. To this solution, 25 ml of dihydropyrane and 25 mg of DL-camphorsulfonic acid are added and allowed to react at 25°C for 6 hours. Monitoring by silica gel thin layer chromatography (chloroform:methanol = 9:1 by volume) reveals the formation of two new compounds having Rf values of 0.32 and 0.44 with the concomitant disappearance of the starting material. The reaction mixture is poured in a mixture of 1 liter acetone and 500 ml water and is kept at pH 11±0.1 under vigorous agitation with 0.5N trisodium phosphate. The two compounds having the said Rf values are found to change slowly to new products having Rf values of 0.12 and 0.23 by thin layer chromatography. After reaction for one hour at 25°C, the solution is neutralized by 1N hydrochloric acid and acetone is removed by evaporation under reduced pressure. The concentrate is extracted with 500 ml and 200 ml of methylene chloride. The methylene chloride extracts are combined; rinsed three times with 500 ml of water; and then dried over anhydrous sodium sulfate. The methylene chloride solution is charged on a column of 50 g silica gel (E. Merck, Darmstadt; Kieselgel 60, 70—230 mesh) and the column is washed with 1 liter of chloroform. Chloroform-methanol mixture is used for elution. Each 20 ml fraction is monitored by thin layer chromatography for product analysis. Fractions containing a product of Rf 0.23 are combined and concentrated under reduced pressure to provide 1.7 g of dark red solid matters of 4'-O-tetrahydropyranyladriamycin *b* (yield 29%). Recrystallization from methylene chloride gives 0.54 g of the above product in the pure state. Melting point under decomposition: 191—192°C.

NMR($CDCl_3$, ppm) 1.33 (6'-H), 1.50~1.96 (tetrahydropyranyl-H), 4.07 (4'-O-methyl-H), 4.73 (14-H), 4.72 (anomeric H of the tetrahydropyranyl group), 5.27 (1'-H), 7.31~8.06 (1~3-H)

IR(KBr, $cm^{-1}$) 3680, 3530, 3390, 1720, 1618, 1578, 1407, 1294, 1209, 1121, 1110, 1075, 1000, 988, 960, 815, 761.

$[\alpha]_D^{19}$ + 202.7° (c=0.75., $CHCl_3$)

Fractions containing the product with Rf 0.13 give 0.65 g of 4'-O-tetrahydropyranyladriamycin *a*.

### Example 2

4'-O-Tetrahydropyranyladriamycins *a* and *b*.

Daunomycin hydrochloride (226 mg) is dissolved in 4 ml of anhydrous methanol and is mixed with 0.2 ml of methyl orthoformate and 8 ml of dioxane. After 0.83 ml of bromine solution in methylene chloride (10% w/v) is added, the reaction is carried out at 25°C for 40 minutes. The reaction mixture is poured in 50 ml of dry ether and the precipitates formed are recovered by centrifugation. The supernatant solution is discarded. After washing twice with 5 ml of dry ether, the precipitates are treated under agitation in 12 ml of acetone at 25°C for one hour. The treated deposits are collected by centrifugation; rinsed twice with ether and dried to give 226 mg of red powder of 14-bromodaunomycin hydrochloride (yield 87%). The red powser is dissolved in 7 ml of dry dimethylformamide and mixed with 2 ml of dihydropyrane and 10 mg of p-toluenesulfonic anhydride. After reaction at 20°C for 3 hours, the reaction mixture is poured in a mixture of 60 ml acetone and 30 ml water and allowed to stand for 30 minutes at pH 11.0—11.5 under control with 1N sodium hydroxide. The solution is neutralized with diluted hydrochloric acid and most of the acetone is evaporated off under reduced pressure. The concentrate is extracted three times with 30 ml of chloroform. The chloroform extracts are combined; rinsed three times with water and dried over anhydrous sodium

sulfate. The chloroform solution is concentrated under reduced pressure to give dark red solid matters. Purification is performed with silica gel thin layer plates (E. Merck, Darmstadt; silica gel 60, 20 × 20 cm, 2 mm thick). Satisfactory separation is obtained by developing with a solvent mixture of chloroform and methanol (9:1). Silica gel powder corresponding to the area of Rf 0.23 is scraped off and eluted with a mixture of chloroform and methanol (2:1). The eluate is concentrated to dryness under reduced pressure to provide 54 mg of reddish brown solid of 4'-O-tetrahydropyranyladriamycin *b* (overall yield 21%).

Similarly 39 mg of 4'-O-tetrahydropyranyladriamycin *a* (overall yield 16%) is recovered from the area of Rf 0.13.

Example 3

4'-O-Tetrahydrofuranyladriamycin

14-Bromodaunomycin hydrochloride (1.10 g) in 30 ml of dry dimethylformamide is mixed with 0.2 ml of dihydrofurane and a small amount of DL-camphorsulfonic acid and then allowed to react at a temperature of 20-25°C for 3 hours. The reaction solution is diluted with a mixture of 200 ml acetone and 100 ml water and stirred vigorously for one hour while the pH of the solution is maintained at 11±0.1 by addition of 0.5 N trisodium phosphate. After neutralization with 1 N hydrochloric acid, the acetone is removed by evaporation *in vacuo*. The concentrate is submitted to extraction twice with 50 ml of methylene chloride and the methylene chloride extracts are combined and rinsed three times with water. The methylene chloride solution is passed through a column of 20 g silica gel (E. Merck, Darmstadt; Silica gel 60) and the adsorbed products are separatedly eluted with a solvent system of chloroform and methanol under monitoring by thin layer chromatography (chloroform:methanol = 9:1 by volume). Fractions containing compounds of Rf 0.16 and 0.19 are collected and concentrated to dryness to give 350 mg of dark red solid of 4'-O-tetrahydrofuranyladriamycin (yield 33%).

Melting point under decomposition: 189—194°C.

NMR($CDCl_3$, ppm) 1.25~1.27 (6'-methyl-H), 1.67~2.30 (tetrahydrofuranyl-H), 4.07 (4-O-methyl-H), 4.75 (14-H), 5.17 and 5.38 (anomeric H of the tetrahydrofuranyl group), 5.30 (1'-H), 7.30~8.07 (1~3-H).

Example 4

Adriamycin hydrochloride

To a solution of 226 mg of daunomycin hydrochloride in 4 ml of anhydrous methanol, 0.2 ml of methyl orthoformate and 8 ml of dioxane are added. After 0.83 ml of brominemethylene chloride solution (10%, w/v) is poured therein, the reaction is carried out at 25°C for 40 minutes. The reaction solution is diluted with 50 ml of dry ether and the formed precipitates are collected by centrifugation, whereas the supernatant is discarded. The precipitates are washed twice with 5 ml of dry ether and then treated under agitation with 12 ml of acetone at 25°C for one hour. After dilution with aqueous acetone, the acetone solution is kept for 20 minutes at pH 11±0.1 by adjusting with 0.5 M sodium phosphate. The treated solution is neutralized and the acetone is evaporated off *in vacuo*. The product is extracted repeatedly with a chloroform-methanol mixture and the extracts are combined and dried over anhydrous sodium sulfate.

After concentration *in vacuo*, adriamycin hydrochloride is forced to precipitate by adding methanolic hydrochloric acid and anhydrous ether to the chloroform concentrate.

Melting point under decomposition: 204~205°C.

$[\alpha]_D^{20}$ + 248° (c=0.1, $CH_3OH$).

TABLE 1

| Compounds | No. | | | Yield | |
|---|---|---|---|---|---|
| 4′-O-(tetrahydro-pyranyl)-ADM | 1—a | —OH | 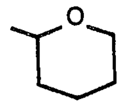 | 16% | See Example 2 |
| „ (b) | 1—b | „ | „ | 21% | |
| 4′-O-(tetrahydro-furanyl)-ADM | 2 | —OH | 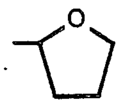 | 46 | |
| 4′-O-(tetrahydro-furanyl)-ADM (a) | 2—a | „ | „ | | |
| „(b) | 2—b | „ | „ | | |
| 4′-O-(6-acetoxy-methyltetrahydro-pyranyl)-ADM | 3 | „ | 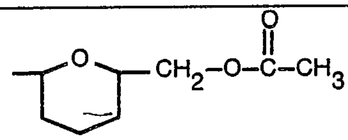 | 37 | |
| 4′-O-(1-ethoxy-ethyl)-ADM (a) | 4—a | „ | 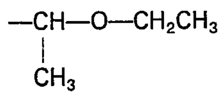 | 25 | |

TABLE 1 — cont'd.

| Compounds | No. | | | Yield |
|---|---|---|---|---|
| 4'-O-(1-ethoxy-ethyl)-ADM (b) | 4—b | —OH | 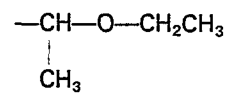 | 24 |
| 4'-O-(1-butyloxy-ethyl)-ADM (a) | 5—a | „ | 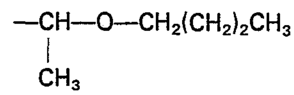 | 14 |
| „ (b) | 5—b | „ | „ | 17 |
| 4'-O-(1-iso-butyloxyethyl)-ADM (a) | 6—a | „ | 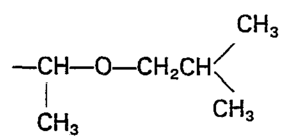 | 18 |
| „ (b) | 6—b | „ | „ | 19 |
| 4'-O-(1-(6-methyl-heptyloxy)ethyl)-ADM (a) | 7—a | „ | 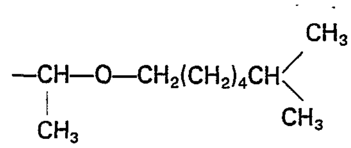 | 15 |
| „ (b) | 7—b | „ | „ | 18 |
| 4'-O-(1-cyclo-hexyloxyethyl)-ADM (a) | 8—a | „ | 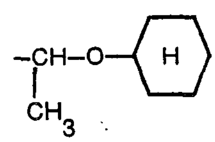 | 16 |
| „ (b) | 8—b | „ | „ | 17 |

**Claims**

1. The compounds of the formula

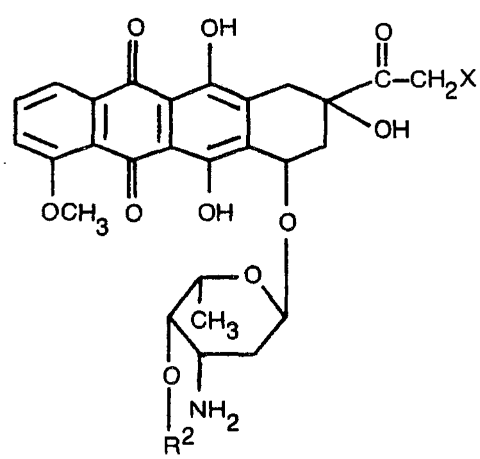

wherein X is a halogen atom and $R^2$ is 6-substituted tetrahydropyran-2-yl especially 6-acetoxymethyltetrahydropyran-2-yl, 6-methoxytetrahydropyran-2-yl, 6-carbomethoxytetrahydropyran-2-yl, tetrahydrofuran-2-yl, alkyloxyethyl, especially ($C_1$ to $C_8$-alkyl)oxyethyl or cyclohexyloxyethyl, and acid addition salts thereof.

2. The compounds of Claim 1 wherein $R^2$ is 6-acetoxymethyltetrahydropyran-2-yl, 6-methoxytetrahydropyran-2-yl, 6-carbomethoxytetra-hydropyran-2-yl, tetrahydrofuran-2-yl, 1-methoxyethyl, 1-ethoxyethyl, 1-butyloxyethyl, 1-isobutyloxyethyl, 1-(6-methylheptyloxy)ethyl or cyclohexyloxyethyl, and acid addition salts thereof.

3. The compounds of claim 1 or 2 wherein X is bromine and acid addition salts thereof.

4. A process for the preparation of compounds of the formula

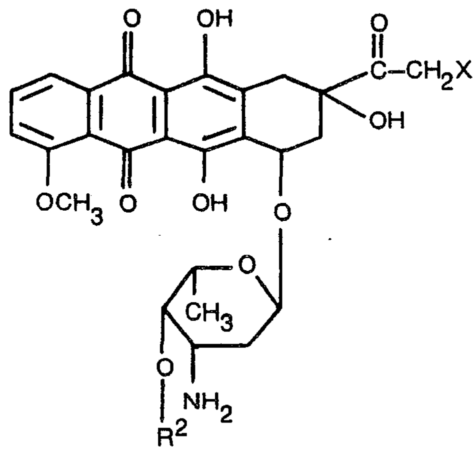

wherein X is a halogen atom and $R^2$ is tetrahydropyran-2-yl, 6-substituted tetrahydropyran-2-yl especially 6-acetoxymethyltetrahydropyran-2-yl, 6-methoxytetrahydropyran-2-yl, 6-carbomethoxytetrahydropyran-2-yl, tetrahydrofuran-2-yl, alkyloxyethyl, especially ($C_1$ to $C_8$-alkyl)oxyethyl or cyclohexyloxyethyl, and acid addition salts thereof, wherein the corresponding compound in which $R^2$ is a hydrogen atom is reacted in the presence of acid catalyst and inert organic solvent with dihydropyrane, an appropriate derivative thereof, dihydrofurane, an appropriate alkyl vinyl ether.

5. A process as claimed in claim 4 wherein the said corresponding compound in which $R^2$ is a hydrogen atom is prepared from daunomycin or a salt thereof by halogenation in the presence of inert organic solvent and treatment with acetone.

6. A process for the preparation of an anthracycline glycoside of the formula

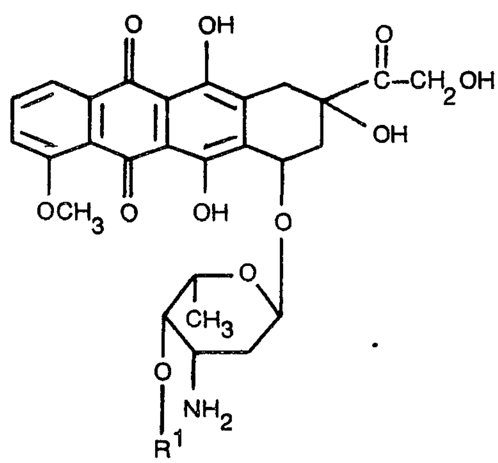

where $R^1$ is 6-substituted tetrahydropyran-2-yl especially 6-acetoxymethyltetrahydropyran-2-yl, 6-methoxytetrahydropyran-2-yl, 6-carbomethoxytetrahydropyran-2-yl, tetrahydrofuran-2-yl, or alkyloxyethyl especially ($C_1$ to $C_8$-alkyl)oxyethyl such as 1-methoxyethyl, 1-ethoxyethyl, 1-butyloxyethyl, 1-isobutyloxyethyl, 1-(6-methylheptyloxy)ethyl and cyclohexyloxyethyl; or an acid addition salt thereof in which a compound as claimed in any of Claims 1 to 3 is hydrolysed.

7. A process as claimed in Claim 6 wherein the hydrolysis is carried out by alkali treatment in lower alcohol and/or aqueous acetone.

8. A process for the preparation of an anthracycline glycoside in accordance with claim 1, 2 or 3, by a threestep process comprising a first step of bromination of a daunomycin acid addition salt, a second step of conversion to 4'-substituted derivatives by a treatment with a hydroxyl reagent to effect pyranylation, furanylation or etherification at the C—4' position, and a third step of hydrolysis.

**Patentansprüche**

1. Verbindungen der Formel

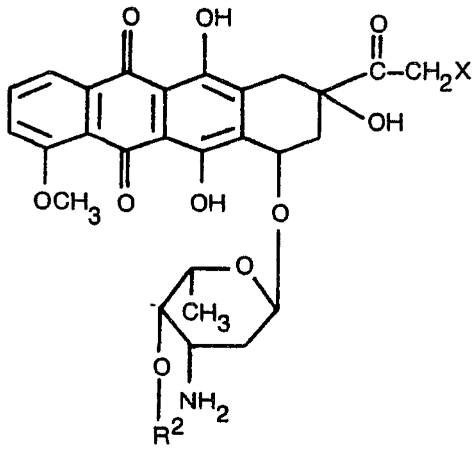

worin X ein Halogenatom ist und $R^2$ 6-substituiertes Tetrahydropyran-2-yl, insbesondere 6-Acetoxymethyltetrahydropyran-2-yl, 6-Methoxytetrahydropyran-2-yl, 6-Carbomethoxytetrahydropyran-2-yl, Tetrahydrofuran-2-yl, Alkyloxyethyl, insbesondere ($C_1$—$C_8$-Alkyl)oxyethyl oder Cyclohexyloxyethyl ist, sowie Säureadditionssalze davon.

2. Verbindungen nach Anspruch 1, worin $R^2$ 6-Acetoxymethyltetrahydropyran-2-yl, 6-Methoxytetrahydropyran-2-yl, 6-Carbomethoxytetra-hydropyran-2-yl, Tetrahydrofuran-2-yl, 1-Methoxyethyl, 1-Ethoxyethyl, 1-Butyloxyethyl, 1-Isobutyloxyethyl, 1-(6-Methylheptyloxy)ethyl oder Cyclohexyloxyethyl ist, sowie Säureadditionssalze davon.

3. Verbindungen nach Anspruch 1 oder 2, dadurch gekennzeichnet, daß X für Brom steht, sowie Säureadditionssalze davon.

4. Verfahren zur Herstellung von Verbindungen der Formel

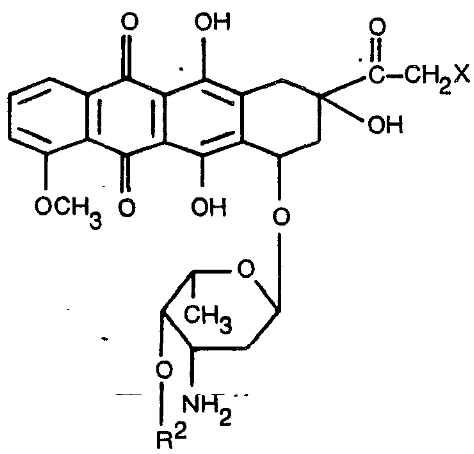

worin X ein Halogenatom ist und $R^2$ Tetrahydropyran-2-yl, 6-substituiertes Tetrahydropyran-2-yl, insbesondere 6-Acetoxymethyltetrahydropyran-2-yl, 6-Methoxytetrahydropyran-2-yl, 6-Carbomethoxytetrahydropyran-2-yl, Tetrahydrofuran-2-yl, Alkoxyoxyethyl, insbesondere ($C_1$—$C_8$-Alkyl)oxyethyl oder Cyclohexyloxyethyl ist, sowie Säureadditionssalzen davon, wobei die entsprechende Verbindung, in welcher $R^2$ ein Wasserstoffatom ist, in Gegenwart eines sauren Katalysators und eines inerten organischen Lösungsmittels mit Dihydropyran, einem geeigneten Derivat davon, Dihydrofuran einem geeigneten Derivat davon oder einem geeigneten Alkylvinylether umgesetzt wird.

5. Verfahren nach Anspruch 4, dadurch gekennzeichnet, daß die entsprechende Verbindung, in der $R^2$ ein Wasserstoffatom ist, hergestellt wird aus Daunomycin oder einem Salz davon durch Halogenierung in Gegenwart eines inerten organischen Lösungsmittels und Behandlung mit Aceton.

6. Verfahren zur Herstellung eines Anthracyclinglycosids der Formel

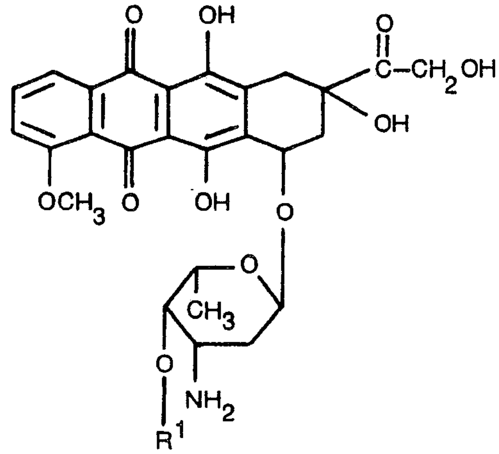

worin $R^1$ 6 substituiertes Tetrahydropyran-2-yl, insbesondere 6-Acetoxymethyltetrahydropyran-2-yl, 6-Methoxytetrahydropyran-2-yl, 6-Carbomethoxytetrahydropyran-2-yl, Tetrahydrofuran-2-yl oder Alkyloxyethyl, insbesondere ($C_1$—$C_8$-Alkyl)oxyethyl, wie 1-Methoxyethyl, 1-Ethoxyethyl, 1-Butyloxyethyl, 1-Isobutyloxyethyl, 1-(6-Methylheptyloxy)ethyl sowie Cyclohexyloxyethyl ist, oder eines Säureadditionssalzes davon, wobei eine Verbindung gemäß einem der Ansprüche 1 bis 3 hydrolysiert wird.

7. Verfahren nach Anspruch 6, dadurch gekennzeichnet, daß die Hydrolyse durch Alkalibehandlung in einem niederen Alkohol und/oder wäßrigen Aceton durchgeführt wird.

8. Verfahren zur Herstellung eines Anthracyclinglykosids gemäß Anspruch 1, 2 oder 3 durch ein 3-Stufenverfahren aus einer ersten Stufe der Bromierung eines Daunomycinsäureadditionssalzes, einer zweiten Stufe der Umwandlung in 4'-substituierte Derivate durch Behandlung mit einem Hydroxylreagens zur Bewirkung einer Pyranylierung, Furanylierung oder Veretherung in der C-4'-Position und einer dritten Stufe einer Hydrolyse.

**Revendications**

1. Les composés de formule

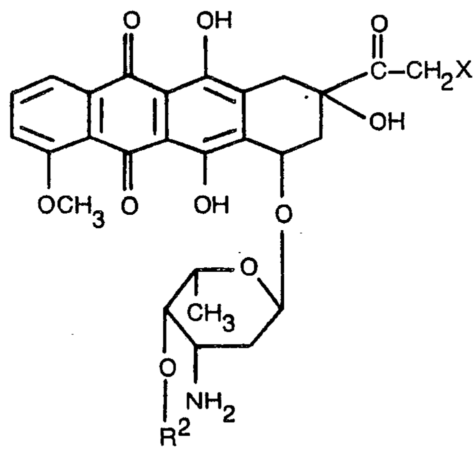

dans laquelle X est un atome d'halogène et $R^2$ est un tétrahydropyranne-2-yle 6-substitué, en particulier un 6-acétoxyméthyltétrahydropyranne-2-yle, un 6-méthoxytétrahydropyranne-2-yle, un 6-carbométhoxytétrahydropyranne-2-yle, un tétrahydrofuranne-2-yle, un alkyloxyéthyle, en particulier un (alkyl en $C_1$ à $C_8$) oxyéthyle ou un cyclohexyloxyéthyle, et leurs sels d'addition d'acides.

2. Les composés de la revendication 1 où $R^2$ est un 6- acétoxyméthyltétrahydropyranne-2-yle, un 6-méthoxytétrahydropyranne-2-yle, un 6-carbométhoxytétrahydropyranne-2-yle, un tétrahydrofuranne-2-yle, un 1-méthoxyéthyle, un 1-éthoxyéthyle, un 1-butoxyéthyle, un 1-isobutoxyéthyle, un 1-(6-méthylheptyloxy)éthyle ou un cyclohexyloxyéthyle, et leurs sels d'addition d'acides.

3. Les composés de la revendication 1 ou 2 où X est un brome et leurs sels d'addition d'acides.

4. Un procédé pour la préparation des composés de formule

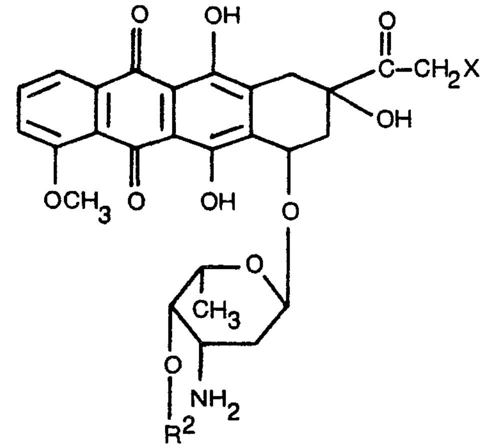

dans laquelle X est un atome d'halogène et $R^2$ est un tétrahydropyranne-2-yle, un tétrahydropyranne-2-yle 6-substitué, en particulier un 6-acétoxyméthyltétrahydropyranne-2-yl, un 6- méthoxytétrahydropyranne-2-yle, un 6-carbométhoxytétrahydropyranne-2-yle, un tétrahydrofuranne-2-yle, un alkyloxyéthyle, en particulier un (alkyl en $C_1$ à $C_8$)oxyéthyle ou un cyclohexyloxyéthyle, et leurs sels d'addition d'acides, dans lequel le composé correspondant où $R^2$ est un atome d'hydrogène est mis à réagir en présence d'un catalyseur acide et d'un solvant organique inerte avec du dihydropyranne, un dérivé approprié de celui-ci, du dihydrofuranne, un dérivé approprié de celui-ci ou un éther d'alkyle et de vinyle approprié.

5. Un procédé comme revendiqué dans la revendication 4 dans lequel ledit composé correspondant où $R^2$ est un atome d'hydrogène est préparé à partir de la daunomycine ou d'un de ses sels par halogénation en présence d'un solvant organique inerte et traitement par l'acetone.

6. Un procédé pour la préparation d'un anthracyclineglycoside de formule

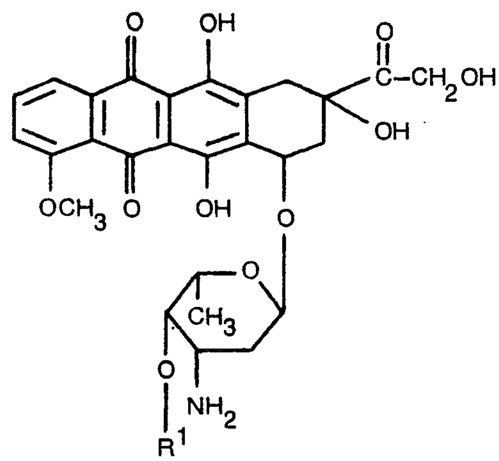

dans laquelle $R^1$ est un tétrahydropyranne-2-yle 6-substitué, en particulier un 6-acétoxyméthyltétrahydropyranne-2-yle, un 6-méthoxytétrahydropyranne-2-yle, un 6-carbométhoxytétrahydropyranne-2-yle, un tétrahydrofuranne-2-yle ou un alkyloxyéthyle, en particulier un (alkyl en $C_1$ à $C_8$)oxyéthyle, tel qu' un 1-méthoxyéthyle, un 1-éthoxyéthyle, un 1-butoxyéthyle, un 1-isobutoxyéthyle, un 1-(6-méthylheptyloxy)éthyle et un cyclohexyloxyéthyle; ou un sel d'addition d'acide de celui-ci, dans lequel on hydrolyse un composé comme revendiqué dans l'une quelconque des revendications 1 à 3.

7. Un procédé comme revendiqué dans la revendication 6 dans lequel l'hydrolyse est effectuée par traitement alcalin dans un alcool inférieur et/ou l'acétone aqueuse.

8. Un procédé pour la préparation d'un anthracyclineglycoside, selon la revendication 1, 2 ou 3, selon un procédé en trois étapes comprenant une première étape de bromation d'un sel d'addition d'acide de la daunomycine, une seconde étape de conversion en dérivés 4'-substitués par un traitement avec un agent réagissant avec un hydroxyle pour effectuer une pyrannylation, une furannylation ou, une éthérification à la position C—4', et une troisième étape d'hydrolyse.